(19) Europäisches Patentamt
European Patent Office
Office européen des brevets

(11) Publication number: 0 127 888 B1

(12) # EUROPEAN PATENT SPECIFICATION

(45) Date of publication of patent specification: 14.01.87

(51) Int. Cl.⁴: C 07 C 46/08, C 07 C 50/02, B 01 J 27/08

(21) Application number: 84106245.8

(22) Date of filing: 31.05.84

(54) **Process for the production of 2,3,5-trimethylbenzoquinone.**

(30) Priority: 06.06.83 JP 100624/83

(43) Date of publication of application: 12.12.84 Bulletin 84/50

(45) Publication of the grant of the patent: 14.01.87 Bulletin 87/03

(84) Designated Contracting States: CH DE FR LI

(58) References cited:
EP-A-0 001 199
FR-A-2 138 030
FR-A-2 245 602
US-A-3 306 875

(73) Proprietor: MITSUBISHI GAS CHEMICAL COMPANY, INC.
5-2, Marunouchi 2-chome Chiyoda-Ku
Tokyo (JP)

(72) Inventor: Isshiki, Tomiya
14-11, 2-chome, Umegaoka
Setagaya-ku Tokyo (JP)
Inventor: Yui, Tomoyuki
No. 14-6, Tokiwadaira-jinyamae
Matsudo-shi Chiba-ken (JP)
Inventor: Uno, Hideo
No. 1-43, Kaname-cho Toshima-ku
Tokyo (JP)
Inventor: Abe, Mitsuo
11-18, 5-chome, Kana-machi Katsushika-ku
Tokyo (JP)

(74) Representative: Türk, Dietmar, Dr. rer. nat. et al
Türk, Gille + Hrabal Patentanwälte Bruckner Strasse 20
D-4000 Düsseldorf 13 (DE)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

Background of the invention

The present invention relates to a process for the production of 2,3,5-trimethylbenzoquinone (hereinafter abbreviated to "TMBQ"), and more particularly, to a process for producing TMBQ by oxidizing a substituted phenol with oxygen in the presence of a catalyst.

TMBQ is a useful substance as an intermediate for use in the preparation of Vitamin E. It is known that TMBQ can be prepared from various starting materials.

As typical methods for the production of TMBQ from a substituted phenol, in particular 2,3,6-trimethylphenol (hereinafter abbreviated to "TMP"), the following are known:

(1) a method of oxidizing TMP with inorganic oxidizing agents; and

(2) a method of oxidizing TMP with oxygen in the presence of a catalyst.

Inorganic oxidizing agents as used in the method (1) include potassium permanganate, manganese dioxide, and lead oxide. The method (1), however, have disadvantages in that it needs stoichiometric amounts of expensive oxidizing agents as described above and it is necessary to treat or regenerate metals in a reduced valency state which result from the oxidation reaction.

As the catalyst for use in the method (2), a cobalt complex catalyst, for example, is known. This method, however, is not suitable for industrial use since the life of the catalyst is very short although its activity is considerably high at an initial stage of the reaction. In addition, there is known a method in which copper halide is used as a catalyst. This method is high in both the yield and selectivity under limited conditions, but there still remain various fundamental problems to be solved in the industrial practice of the method.

Japanese Patent Publication No. 17585/1978, for example, discloses a method of oxidizing TMP with oxygen by the use as a catalyst of copper ion and halogen ion. Although the method is superior in that the yield is high under limited conditions, it has disadvantages in that the rate of reaction is low, the influences of water on the reaction are very serious, and in that a large amount of energy is expected to be consumed in the isolation of TMBQ or reuse of the catalyst. When the catalyst is intended to recover and reuse after the reaction is completed, it is necessary that the reaction solution be extracted with a large amount of water, the resulting aqueous solution be freed of water by techniques such as evaporation, and the catalyst be recovered in a water-free condition. In industrially recovering and reusing the catalyst, a large amount of energy is consumed, which is seriously disadvantageous from an economic standpoint. Furthermore, since the catalyst is of low activity, the method has disadvantages in that the reaction time is long, the space time yield is low, and the reactor is required to be increased in size. Thus, many difficulties are encountered in the industrial practice of the method.

Japanese Patent Application Laid-Open No. 93931/1975 discloses an improved method over the method of Japanese Patent Publication No. 17585/1978. This method improves adverse influences of water exerted on the reaction, allows the reaction to efficiently proceed even in water-containing solvents, and makes it easy to repeatedly use the catalyst. This method is intended to prevent deterioration of the catalyst and to repeatedly use copper halide dissolved in an aqueous phase as a catalyst without application of any treatment, by adding to the reaction system, in advance or intermittently, halogen or halogenated compounds capable of releasing halogen in the reaction system, such as bromine, chlorine, hydrogen halide, hypohalogenous acid salts, and 4-bromo-2,3,6-trimethylphenol. As apparent from the examples as described hereinafter, such halogen compounds are consumed and diminished at a considerably earlier stage of repeated uses. It is necessary, therefore, to supplement the halogen compounds from time to time. This is troublesome in the operation and will increase the production costs in the industrial production of TMBQ.

In FR—A—2 245 602 there is disclosed a process for producing benzoquinone or substituted benzoquinone by oxidizing phenol or substituted phenol with oxygen in the presence of a catalyst. As catalyst a copper halide is used. However, the reaction speed is very low in this known process.

Furthermore, in all the known techniques, it is essential to use organic solvents which are completely soluble in water. Therefore, in the separation of the desired TMBQ after the reaction, the operation of separating TMBQ from the catalyst and solvent becomes complicated. Furthermore, other operations such as concentration, dehydration and purification are needed in repeated use of the catalyst and solvent. This is expected to increase the amount of energy consumed.

Summary of the invention

The present invention is intended to overcome the above-described problems of the prior art, and the object of the present invention is to provide a process for the production of TMBQ which is greatly superior to conventional processes.

The present invention, in an embodiment, relates to a process for producing 2,3,5-trimethylbenzoquinone which comprises contacting 2,3,6-trimethylphenol with molecular oxygen in a medium consisting of water and an aliphatic alcohol having from 4 to 10 carbon atoms in the presence as a catalyst of a copper halogeno complex having the following general formula:

$$M_l[Cu(II)_mX_n]_p \qquad (I)$$

wherein M is an alkali metal or ammonium, Cu(II) is a divalent copper, X is a halogen atom, l is an integer of from 1 to 3, m is 1 or 2, n is an integer of from 3 to 8, p is 1 or 2, and $l+2mp=np$.

The copper halogeno complex may or may not contain crystal water.

In another embodiment, the present invention relates to a process for producing 2,3,5-trimethylbenzoquinone which comprises contacting 2,3,6-trimethylphenol with molecular oxygen in a medium consisting of water and an aliphatic alcohol having from 4 to 10 carbon atoms in the presence as a catalyst of a copper halogeno complex of the same general formula as described above and an alkali metal halide.

## Brief description of the drawings

Fig. 1 is a graph showing the relation between the concentration of a copper halogeno complex and its visible absorption spectrum;

Fig. 2 is a graph showing the relation between the concentration of a copper halogeno complex and a molar absorption coefficient of visible absorption spectrum.

Fig. 3 is a graph showing the relation between the concentration of halogen and a molar absorption coefficient of visible absorption spectrum.

## Detailed description of the invention

The copper halogeno complexes represented by the general formula as described above are used as catalysts in the present invention. These copper halogeno complexes accelerate oxidation of TMP with oxygen and permit to convert TMP into TMBQ with high selectivity. The copper halogeno complex catalyst of the present invention is superior in the rate of reaction and selectivity of TMBQ to the known catalyst system comprising free copper and halogen ions even in the reaction system where water is present and, furthermore, the drop in the activity of the catalyst due to water is scarcely observed.

The copper halogeno complex catalyst of the present invention is a novel catalyst, and produces very favorable effects in the industrial practice of the process of the present invention; for example, the size of a reactor can be greatly reduced, the space time yield can be increased, and the catalyst can be used repeatedly.

The copper halogeno complex catalyst of the present invention has excellent properties that even when used singly, it permits easy oxidation of TMP with oxygen and converts TMP into TMBQ with high selectivity. When, however, the copper halogeno complex catalyst is used in combination with alkali metal halides, the selectivity of TMBQ is more increased and, furthermore, the rate of reaction is increased.

The copper halogeno complexes as used herein are coordination compounds of copper and halogen, and are represented by the general formula:

$$M_l[Cu(II)_mX_n]_p \qquad (I)$$

wherein M is an alkali metal or ammonium, Cu(II) is a divalent copper, X is a halogen atom, l is an integer of from 1 to 3, m is 1 or 2, n is an integer of from 3 to 8, p is 1 or 2, and $l+2mp=np$. They may or may not contain crystal water.

The alkali metal includes lithium (Li), potassium (K), rubidium (Rb), and cesium (Cs). Of these metals, lithium, potassium, and cesium are preferred. Particularly preferred is lithium. The halogen is preferably chlorine (Cl), bromine (Br), and iodine (I). Particularly preferred are chlorine and bromine. Typical examples of the copper halogeno complexes are shown below:

$Li[CuCl_3]\cdot 2H_2O$, $NH_4[CuCl_3]\cdot 2H_2O$, $(NH_4)_2[CuCl_4]\cdot 2H_2O$, $K[CuCl_3]$, $K_2[CuCl_4]\cdot 2H_2O$, $Cs[CuCl_3]\cdot 2H_2O$, $Cs_2[CuCl_4]\cdot 2H_2O$, $Cs_3[Cu_2Cl_7]\cdot 2H_2O$, $Li_2[CuBr_4]\cdot 6H_2O$, $K[CuBr_3]$, $(NH_4)_2[CuBr_4]\cdot 2H_2O$, $Cs_2[CuBr_4]$, and $Cs[CuBr_3]$.

These copper halogeno complexes can be prepared by known procedures such as those methods described in *Mellor's Comprehensive Treatment on Inorganic and Theoretical Chemistry*, Vol. III, pages 182—201 (Longman).

The thus-prepared copper halogeno complexes can be identified by known techniques such as by measuring their melting points. For example, the copper chloride-lithium complex, $Li[CuCl_3]\cdot 2H_2O$, is red brown in color and is different in appearance from cupric chloride, $CuCl_2\cdot 2H_2O$, which is a green crystal, and the melting point of the complex is 130—135°C. According to the above-described reference, *Mellor's Comprehensive Treatment on Inorganic and Theoretical Chemistry*, Vol. III, pages 184 and 169 (Longman), the melting points of a copper chloride-lithium complex, $Li[CuCl_3]\cdot 2H_2O$, and cupric chloride, $CuCl_2\cdot 2H_2O$, are 130°C and 110°C, respectively.

In visible absorption spectra of an aqueous copper halogeno complex solution and an aqueous cupric chloride solution, the maximum absorption peaks are between 820 and 880 nm. As the concentration of the copper halogeno complex or cupric chloride becomes higher, the maximum absorption peak shifts to the longer wavelength side (Fig. 1). Wavelengths at which the maximum absorption peak appears are measured for the copper halogeno complex and cupric chloride at the same concentration and have been proved to be materially different from each other. When the molar absorption coefficient at a wavelength of

800 nm is plotted against the concentration, the molar absorption coefficient being plotted on a logarithmic scale (log ε), it can be seen that the molar absorption coefficient for cupric chloride saturates at concentrations of 3 moles per liter (mol/l) or more, the maximum molar absorption coefficient being 1.45, whereas the molar absorption coefficient for $Li[CuCl_3] \cdot 2H_2O$, for example, saturates at concentrations of 3 mol/l or more, the maximum molar absorption coefficient being 1.52; that is, their molar absorption coefficients are materially different from each other (Fig. 2).

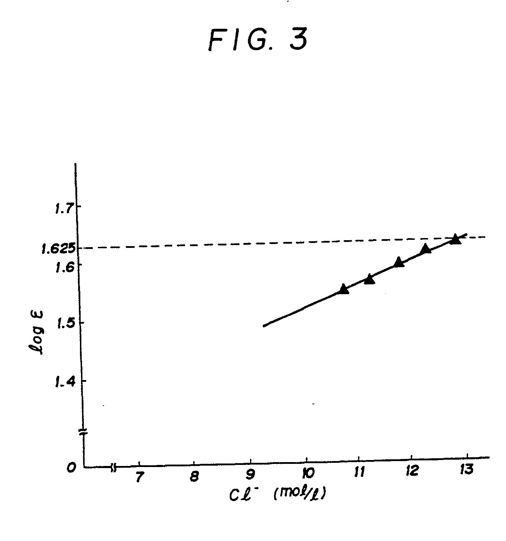

When lithium chloride is dissolved in a saturated solution of $Li[CuCl_3] \cdot 2H_2O$, the molar absorption coefficient approaches 1.63 (Fig. 3). When a combination of a copper chloride lithium complex, $Li[CuCl_3] \cdot 2H_2O$, and lithium chloride is used, it is preferred that the molar absorption coefficient (log ε) of an aqueous solution of the combination be from 1.4 to 1.63.

The pH of an aqueous copper halogeno complex solution varies depending on the concentration of the copper halogeno complex but usually is 3.0 or less. Surprisingly it has been found that if an alkali metal halide is dissolved in the aqueous copper halogeno complex solution, the pH value further drops almost in proportion to the amount of the alkali metal halide added. This tendency does not change even if an alcohol is added to the solution.

The pH of an aqueous solution of the copper halogeno complex catalyst of the present invention is usually from 2 to 3 and preferably from 2 to 2.5. In a case where an alkali metal halide is used in combination, the pH of an aqueous solution of the catalyst of copper halogeno complex and alkali metal halide in the present invention is usually 2 or less and preferably from 2 to −1, with the range of from 1 to −0.5 being most preferred.

Alkali metal halides which can be used in combination with the copper halogeno complexes of the present invention are shown below:

NaCl, LiCl, KCl, CsCl, NaBr, $NH_4Br$, KBr, CsBr, NaI, LiI, KI, CsI, etc.

Of these compounds, lithium chloride (LiCl) is especially preferred from viewpoints of yield and a rate of reaction. The catalyst composed mainly of the copper halogeno complex and the alkali metal halide is hereinafter referred to as "copper halogeno complex/alkali metal halide catalyst".

In the practice of the process of the present invention, in general, a copper halogeno complex or a mixture of a copper halogeno complex and an alkali metal halide is dissolved in a predetermined amount of water in a predetermined proportion to prepare a copper halogeno complex or copper halogeno complex/alkali metal halide catalyst of the present invention, an organic solvent and TMP are added, and then TMP is contacted with oxygen in the presence of the catalyst.

In cases where alkali metal halides are used in combination, the ratio of alkali metal halide to copper halogeno complex in the copper halogeno complex/alkali metal halide catalysts is one of the significant factors exerting influences on the oxidation of TMP. In general, if the ratio of alkali metal halide to copper halogeno complex is too large, a rate of reaction is undesirably reduced, whereas if it is too small, the effect of addition of alkali metal halides cannot be obtained, although it varies depending on the type of the copper halogeno complex and alkali metal halide. In addition, the proportion of the alkali metal halide used is limited by its solubility in the reaction system. Thus, although the molar ratio of alkali metal halide to copper halogeno complex cannot be determined unconditionally, it is usually from 1 to 15, preferably from 1 to 10, and most preferably from 2 to 5.

In the present invention, the concentration of the copper halogeno complex in an aqueous phase of the reaction system exerts great influences on the yield and rate of reaction. That is, the amount of water added to the reaction system is one of the most important factors to efficiently carry out the reaction. From a viewpoint of efficiency of the reaction, it is preferred that the concentration of the copper halogeno complex/alkali metal halide catalyst in the aqueous phase is high. If the concentration of the copper halogeno complex in the aqueous phase is 20% by weight or less, the selectivity of TMBQ seriously drops. The concentration of the copper halogeno complex in the aqueouse phase (excluding alcohols), taking into consideration its solubility, is preferably from 20 to 70% by weight, more preferably from 20 to 60% by weight, and most preferably from 30 to 60% by weight.

In the case of the copper halogeno complex/alkali metal halide catalyst, the concentration of the mixture of the copper halogeno complex and the alkali metal halide in the aqueous phase of the reaction system also exerts great influences on the yield and rate of reaction. That is, also in this case, the amount of water added to the reaction system is one of the most important factors to efficiently carry out the reaction of the present invention. From a viewpoint of efficiency of the reaction, it is preferred that the concentration of the copper halogeno complex/alkali metal halide catalyst be as high as possible. If the concentration of the mixture of the copper halogeno complex and the alkali metal halide in the aqueous phase is 20% by weight or less, the selectivity of TMBQ seriously drops. The concentration of the mixture of the copper halogeno complex and the alkali metal halide in the aqueous phase (excluding alcohols), taking into consideration also its solubility, is preferably from 20 to 80% by weight, more preferably from 20 to 70% by weight, and most preferably from 20 to 60% by weight.

The optimum amount of the copper halogeno complex used is determined by both the yield and rate of reaction. That is, there is a tendency that if the amount of the copper halogeno complex used is too small, the yield of TMBQ is small and the rate of reaction is also low. On the other hand, even if the copper

halogeno complex is used in amounts in excess of a certain level, no additional effect can be obtained and, therefore, addition of such large amounts of the copper halogeno complex is of no value. The amount of the copper halogeno complex used in the present invention varies with the type of the copper halogeno complex and cannot be determined unconditionally. In general, in the case of the copper halogeno complex catalyst, the copper halogeno complex is used in an amount of from 0.1 to 5 moles, preferably from 0.5 to 3 moles, and most preferably from 1 to 2 moles per mole of TMP. This can be applied also to the copper halogeno complex/alkali metal halide catalyst.

The medium as used herein is heterogeneous since the alcohols having from 4 to 10 carbon atoms are immiscible or little miscible with water. Thus the process of the present invention is carried out in a heterogeneous reaction system. In accordance with the present invention, therefore, the catalyst can be recycled and used repeatedly, and the reaction product can be separated efficiently.

The alcohols as used herein are aliphatic alcohols having from 4 to 10 carbon atoms. Taking into consideration solubility in water, it is preferred to use aliphatic alcohols having from 5 to 10 carbon atoms. Preferred examples of aliphatic alcohols which can be used in the present invention include n-butanol, n-amyl alcohol, isoamyl alcohol, n-hexyl alcohol, 2-ethylhexanol, n-heptyl alcohol, n-octyl alcohol, n-nonyl alcohol, and n-decyl alcohol.

The medium as used herein is effective in dissolving the copper halogeno complex or both copper halogeno complex and alkali metal halide, dissolving TMP as a starting material, and also in dissolving oxygen. Thus, the desired TMBQ can be produced very efficiently only by contacting these solutions.

When, in particular, aliphatic alcohols having from 5 to 10 carbon atoms are used, the reaction system is completely heterogeneous. Surprisingly, however, it has been found that the reaction proceeds efficiently even in such a heterogeneous condition. After the reaction is completed, the reaction mixture is composed of an aqueous phase as a catalyst layer and an organic phase as an alcohol layer containing TMBQ. Thus the catalyst and TMBQ can be easily separated by applying phase separation. This is very advantageous since post treatments for reuse of the catalyst and recovery of TMBQ can be very simplified.

The amount of the alcohols used is determined taking into consideration the dissolution of TMP and the ratio of alcohols to water. The weight ratio of alcohols to water is from 0.5 to 10, preferably from 0.8 to 5, and most preferably from 1.5 to 2. In other words, the weight ratio of water to alcohols is from 0.1 to 2, preferably from 0.2 to 1.25, and most preferably from 0.5 to 0.67.

The reaction temperature varies over wide ranges depending on the type and amount of each of the copper halogeno complex or copper halogeno complex and alkali metal halide, the amount of water used, and the type of the alcohol. In general, the reaction temperature is preferably from 10 to 120°C, more preferably from 30 to 100°C, and most preferably from 40 to 80°C. The reaction time also varies over wide ranges depending on the type and amount of the copper halogeno complex and alkali metal halide, the reaction temperature, the amount of water used, the type of the organic solvent, and so forth. In general, the reaction time is from about 5 to about 300 minutes.

TMP used as a starting material in the present invention is not limited in its method of preparation. For example, TMP prepared by alkylation of phenols (e.g. alkylation of m-cresol, 2,3-xylenol, and 2,5-xylenol), transalkylation of polymethyl phenol, alkali-fusion of 2,3,6-trimethylbenzenesulfonic acid, oxidation of 2,3,6-trimethylcumene, fractionation of tars, and by isolation from phenols can be used.

The oxygen as used herein includes pure oxygen and oxygen-containing gases. These oxygen-containing gases include oxygen-rich air, air, and oxygen diluted with inert gas. Inert gases which can be used to dilute oxygen include nitrogen, helium and argon. The reaction pressure is, as an oxygen partial pressure, from 0.05 to 50 bar ($kg/cm^2$), preferably from 0.1 to 20 bar ($kg/cm^2$), more preferably from 0.2 to 10 bar ($kg/cm^2$), more preferably from 0.3 to 3 bar ($kg/cm^2$) and most preferably from 0.3 to 1 bar ($kg/cm^2$) (absolute pressure).

TMBQ is produced by contacting TMP with oxygen in the presence of the copper halogeno complex or copper halogeno complex/alkali metal halide catalyst in the mixed solvent of water and alcohols. In the most preferred embodiment of the present invention, an agitation-type reactor is used, since it permits efficient contact between gas and liquid.

TMP can be contacted with oxygen by techniques such as a method of passing oxygen through the reactor and a gas circulation method in which oxygen absorbed is compensated by supplying fresh oxygen to maintain the pressure at a given level.

The process of the present invention can be carried out in a batch system, a semi-batch system, or in a continuous flow system.

The process of the present invention produces various advantages. For example, since copper halogeno complexes as defined above are used as the catalyst, TMP can be easily oxidized with oxygen and TMBQ can be formed in high yields and high selectivities. Addition of alkali metal halides as also defined above permits to more increase the selectivity and to increase the yield by about 10%.

Irrespective of the fact that the process of the present invention is carried out in a reaction system containing water, the process of the present invention is superior in the rate of reaction and selectivity of TMBQ to conventional processes using the known catalyst system comprising free copper and halogen ions. Furthermore, the process of the present invention has a surprising feature that the drop in the activity of the catalyst due to water is scarcely observed. Thus, the process of the present invention is very useful for use in industrial production of TMBQ from TMP.

TMBQ, the product of the present invention, can be easily separated and recovered. The reason is, as described above, that since aliphatic alcohols having from 4 to 10 carbon atoms immiscible or little miscible with water are used, the reaction mixture can be easily separated into the aqueous phase, i.e., catalyst layer, and the organic phase, i.e., alcohol layer containing TMBQ. This separation can be made easier by using aliphatic alcohols having from 5 to 10 carbon atoms. Therefore, TMBQ can be obtained by distilling away the alcohol from the alcohol layer, and the catalyst layer can be used repeatedly, as such or if necessary, after concentration or purification. Alcohols used in the present invention can be easily available at a low price. Thus the process of the present invention is industrially greatly superior to conventional processes.

On reducing the alcohol layer as such, a precursor for Vitamin E, 2,3,5-trimethylhydroxyquinone, can be obtained.

The present invention is described in greater detail with reference to the following Examples and Comparative Examples. In these examples, all the conversions and yields are indicated in moles.

Examples 1 to 8 and Comparative Example 1

A 100-milliliter four-necked flask was charged with 3.4 grams (25 millimoles) of TMP, 4.3 grams (25 millimoles) of $CuCl_2 \cdot 2H_2O$ or 25 millimoles of a previously prepared copper halogeno complex as shown in Table 1, 10 milliliters of n-butyl alcohol, and a predetermined amount of water as shown in Table 1.

After replacement of the atmosphere in the flask with oxygen, the mixture was maintained at 60°C by heating or cooling from outside and vigorously stirred at 800 revolutions per minute (rpm). Oxygen was successively introduced into the flask from a gas holder, and the amount of oxygen consumed was measured by means of a gas biuret. When the absorption of oxygen gas stopped, the reaction was taken as completed.

The alcohol layer, after separation from the water layer, was analyzed by gas chromatography. The results are shown in Table 1.

TABLE 1

| Run No. | Catalyst | Amount of water (milliliters) | Rate of absorption of oxygen (% per hour) | Conversion of TMP (%) | Yield of TMBQ (%) |
|---|---|---|---|---|---|
| 1 (Example 1) | Complex A | 10 | 250 | 100 | 80.5 |
| 2 (Example 2) | Complex B | 10 | 150 | 100 | 79.9 |
| 3 (Example 3) | Complex C | 10 | 110 | 100 | 83.1 |
| 4 (Example 4) | Complex D | 10 | 90 | 100 | 79.6 |
| 5 (Example 5) | Complex E | 15 | 91 | 100 | 82.8 |
| 6 (Example 6) | Complex F | 10 | 87 | 100 | 79.1 |
| 7 (Example 7) | Complex G | 15 | 85 | 100 | 82.0 |
| 8 (Example 8) | Complex H | 15 | 88 | 100 | 82.7 |
| 9 (Comparative Example 1) | $CuCl_2 \cdot 2H_2O$ | 10 | 80 | 100 | 69.5 |

Note:

Complex A: $Li[CuCl_3] \cdot 2H_2O$
Complex B: $(NH_4)[CuCl_3] \cdot 2H_2O$
Complex C: $(NH_4)_2[CuCl_4] \cdot 2H_2O$
Complex D: $K[CuCl_3]$
Complex E: $K_2[CuCl_4] \cdot 2H_2O$
Complex F: $Cs[CuCl_3]$
Complex G: $Cs_2[CuCl_4]$
Complex H: $Cs_2[CuBr_4]$.

$$\text{Rate of absorption of oxygen} \ldots \frac{\text{Amount of oxygen absorbed per hour}}{\text{Theoretical amount of oxygen absorbed}} \times 100$$

Examples 9 to 11

The procedure of Example 1 was repeated wherein 3.4 grams (25 millimoles) of TMP, 25 millimoles of a

previously prepared copper halogeno complex as shown in Table 2, 10 milliliters of n-hexyl alcohol, and 10 milliliters of water were used.

The results are shown in Table 2.

After the reaction, the reaction mixture separated into a water layer and an alcohol layer. The water layer (catalyst layer), after separation from the alcohol layer, could be recycled and reused.

TABLE 2

| Run No. | Copper halogeno complex | Amount of water (milliliters) | Rate of absorption of oxygen (% per hour) | Conversion of TMP (%) | Yield of TMBQ (%) |
|---|---|---|---|---|---|
| 1 (Example 9) | Complex B | 10 | 170 | 100 | 81.5 |
| 2 (Example 10) | Complex E | 15 | 110 | 100 | 83.0 |
| 3 (Example 11) | Complex G | 15 | 87 | 100 | 83.5 |

Note:
Complexes B, E and G, and rate of absorption of oxygen: same as described in Table 1.

Examples 12 and 13

The procedure of Example 1 was repeated wherein 3.4 grams (25 millimoles) of TMP, a predetermined amount of a previously prepared copper halogeno complex, $Li[CuCl_3]\cdot 2H_2O$, as shown in Table 3, 10 milliliters of water, and 10 milliliters of n-hexyl alcohol were used.

The results are shown in Table 3.

After the reaction, the reaction mixture separated into a water layer and an alcohol layer. The water layer (catalyst layer), after separation from the alcohol layer, could be recycled and reused.

TABLE 3

| Run No. | Complex (grams (millimoles)) | Rate of absorption of oxygen (% per hour) | Conversion of TMP (%) | Yield of TMBQ (%) |
|---|---|---|---|---|
| 1 (Example 12) | 6.6 (50) | 230 | 100 | 84.1 |
| 2 (Example 13) | 15.9 (75) | 130 | 100 | 83.5 |

Note:
Rate of absorption of oxygen ... same as described in Table 1.

Examples 14 to 18

The procedure of Example 1 was repeated wherein 3.4 grams (25 millimoles) of TMP, 5.3 grams (25 millimoles) of a copper halogeno complex, $Li[CuCl_3]\cdot 2H_2O$, 10 milliliters of water, and 10 milliliters of an alcohol as shown in Table 4 were used.

The results are shown in Table 4.

After the reaction, the reaction mixture separated into a water layer and an alcohol layer. The water layer (catalyst layer), after separation from the alcohol layer, could be recycled and reused.

TABLE 4

| Run No. | Alcohol | Rate of absorption of oxygen (% per hour) | Conversion of TMP (%) | Yield of TMBQ (%) |
|---|---|---|---|---|
| 1 (Example 14) | n-Butyl alcohol | 250 | 100 | 80.5 |
| 2 (Example 15) | i-Amyl alcohol | 230 | 100 | 80.1 |
| 3 (Example 16) | n-Hexyl alcohol | 230 | 100 | 80.3 |
| 4 (Example 17) | n-Octyl alcohol | 230 | 100 | 80.4 |
| 5 (Example 18) | n-Decyl alcohol | 200 | 100 | 80.0 |

Note:
Rate of absorption of oxygen ... same as described in Table 1.

Examples 19 to 21

The procedure of Example 1 was repeated wherein 3.4 grams (25 millimoles) of TMP, 5.3 grams (25

millimoles) of a copper halogeno complex, Li[$CuCl_3$] · $2H_2O$, 10 milliliters of water, and 10 milliliters of n-butyl alcohol were used and the reaction temperature was changed as shown in Table 5.

The results are shown in Table 5.

After the reaction, the reaction mixture separated into a water layer and an alcohol layer. The water layer (catalyst layer), after separation from the alcohol layer, could be recycled and reused.

TABLE 5

| Run No. | Reaction temperature (°C) | Time required till gas absorption stops (minutes) | Conversion of TMP (%) | Yield of TMBQ (%) |
|---|---|---|---|---|
| 1 (Example 19) | 80 | 30 | 100 | 78.6 |
| 2 (Example 20) | 60 | 40 | 100 | 80.5 |
| 3 (Example 21) | 40 | 240 | 100 | 75.2 |

Example 22

A 200-milliliter four-necked flask was charged with 6.8 grams (50 millimoles) of TMP, 10.6 grams (50 millimoles) of a copper halogeno complex, Li[$CuCl_3$] · $2H_2O$, 20 milliliters of n-octyl alcohol, and 20 milliliters of water. The reaction was performed for 1 hour in the same manner as in Example 1. After the reaction was completed, the reaction mixture was separated into an organic layer and a water layer. The organic layer was analyzed by gas chromatography to determine the conversion of TMP and yield of TMBQ. The water layer was returned to the four-necked flask. Then, 6.8 grams of TMP and n-octyl alcohol were introduced in the four-necked flask and reacted. This operation was repeated several times and deterioration of the catalyst was examined.

The results are shown in Table 6.

TABLE 6

| Run No. | Number of operations repeated | Conversion of TMP (%) | Yield of TMBQ (%) |
|---|---|---|---|
| 1 | 1 | 100 | 76.1 |
| 2 | 2 | 99.9 | 80.3 |
| 3 | 3 | 99.9 | 75.1 |
| 4 | 4 | 99.8 | 78.3 |
| 5 | 5 | 99.8 | 79.1 |
| 6 | 6 | 99.8 | 80.2 |
| 7 | 7 | 99.8 | 80.5 |
| 8 | 8 | 100 | 80.8 |
| 9 | 9 | 99.8 | 78.1 |
| 10 | 10 | 99.8 | 78.0 |
| 11 | 11 | 99.9 | 79.6 |

Comparative Example 2

A mixture of 25 millimoles of a copper chloride/lithium complex, Li[$CuCl_3$] · $2H_2O$, 10 milliliters of water, 10 milliliters of n-butanol, and 2.4 grams (25 millimoles) of phenol was placed in a four-necked flask and reacted with stirring under the same conditions as in Example 1. Four hours after the start of the reaction, the reaction mixture was treated in the same manner as in Example 1 and analyzed by gas chromatography.

The results were as follows:

| | |
|---|---|
| Conversion of phenol | 82% |
| Yield of o-chlorophenol | 3.2% |
| Yield of p-chlorophenol | 26.8% |
| Yield of dichlorophenol | 0.8% |

Any other compound could not be detected by the gas chromatography analysis.

Examples 23 to 26, Reference Examples 1 to 4, and Comparative Example 3

A mixture of 25 millimoles of a previously prepared crystalline copper halogeno complex or commercially available copper halogeno complex and 75 millimoles of an alkali metal halide was placed in a four-necked flask. Then, 10—20 milliliters of water was added to the mixture which was then stirred to prepare a catalyst solution.

Thereafter, 3.4 grams (25 millimoles) of TMP and 10 milliliters of n-octyl alcohol were introduced into the flask.

After replacement of the atmosphere in the flask with oxygen, the mixture was maintained at 60°C and vigorously stirred at 800 rpm. Oxygen was successively introduced from a gas holder and the amount of oxygen consumed was measured by the use of a gas biuret. When absorption of oxygen stopped, the reaction was taken as completed. After the reaction was completed, the reaction mixture was separated into an organic layer and a water layer. The organic layer was analyzed by gas chromatography.

The results are shown in Table 7.

TABLE 7

| Run No. | Catalyst | | Amount of water (milliliters) | Conversion of TMP (%) | Yield of TMBQ (%) | Yield of HMBP (%) | Rate of absorption of oxygen (% per hour) |
|---|---|---|---|---|---|---|---|
| | Complex | Chloride | | | | | |
| 1 (Example 23) | A | LiCl | 10 | 100 | 95 | 4 | 370 |
| 2 (Reference Example 1) | A | — | 10 | 100 | 80 | 15 | 250 |
| 3 (Example 24) | B | LiCl | 10 | 100 | 85 | 5.4 | 180 |
| 4 (Reference Example 2) | B | — | 10 | 100 | 80 | 12 | 150 |
| 5 (Example 25) | C | KCl | 15 | 100 | 83 | 9 | 130 |
| 6 (Reference Example 3) | C | — | 15 | 100 | 79 | 11 | 110 |
| 7 (Example 26) | D | CsCl | 20 | 100 | 85 | 9 | 95 |
| 8 (Reference Example 4) | D | — | 20 | 100 | 81 | 13 | 90 |
| 9 (Comparative Example 3) | — | $CuCl_2$ | 10 | 100 | 69.1 | 18 | 80 |

Note:

Complexes A to D . . . same as described in Table 1.
$CuCl_2$ . . . $CuCl_2$/TMP (molar ratio)=1/1
HMBP . . . Hexamethylbiphenol
Rate of absorption of oxygen . . . same as described in Table 1.

Examples 27 to 35

The procedure of Example 1 was repeated wherein 25 millimoles of TMP, predetermined amounts of a copper halogeno complex, $Li[CuCl_3] \cdot 2H_2O$, and LiCl as shown in Table 8, 10 milliliters of n-octyl alcohol, and 10 milliliters of water were used.

The results are shown in Table 8.

After the reaction, the reaction mixture separated into a water layer and an alcohol layer. The water layer (catalyst layer), after separation from the alcohol layer, could be recycled and reused.

TABLE 8

| Run No. | $Li(CuCl_3)/LiCl/TMP$ (molar ratio) | Conversion of TMP (%) | Yield of TMBQ (%) | Yield of HMBP (%) | Yield of 4-Cl-TMP (%) | Rate of absorption of oxygen (% per hour) |
|---|---|---|---|---|---|---|
| 1 (Example 27) | 0.14/0.14/1 | 100 | 85 | 8 | 1 | 90 |
| 2 (Example 28) | 0.14/0.7/1 | 100 | 81 | 8 | 4 | 85 |
| 3 (Example 29) | 0.5/3.5/1 | 100 | 89 | 7 | Trace | 180 |
| 4 (Example 30) | 0.5/7.5/1 | 100 | 87 | 3 | Trace | 95 |
| 5 (Example 31) | 1/5/1 | 100 | 96 | 3 | Trace | 310 |
| 6 (Example 32) | 1/7/1 | 100 | 96 | 1 | Trace | 100 |
| 7 (Example 33) | 2.5/4.5/1 | 100 | 92 | 8 | Trace | 90 |
| 8 (Example 34) | 2.5/7.5/1 | 100 | 95 | 4 | Trace | 90 |
| 9 (Example 35) | 2.5/12.5/1 | 100 | 96 | 3 | Trace | 90 |

Note:
Rate of absorption of oxygen . . . same as defined in Table 1.

Examples 36 to 38

The procedure of Example 1 was repeated wherein 25 millimoles of TMP, 25 millimoles of a previously prepared crystalline complex, $Li[CuCl_3] \cdot 2H_2O$, 75 millimoles of LiCl, and 10 milliliters of n-hexanol were used and the amount of water added was changed as shown in Table 9.

The results are shown in Table 9.

After the reaction, the reaction mixture separated into a water layer and an alcohol layer. The water layer (catalyst layer), after separation from the alcohol layer, could be recycled and reused.

TABLE 9

| Run No. | Amount of water used (grams) | Concentration of catalyst (% by weight) | pH of catalyst layer | Conversion of TMP (%) | Yield of TMBQ (%) | Yield of HMBP (%) | Rate of absorption of oxygen (% per hour) |
|---|---|---|---|---|---|---|---|
| 1 (Example 36) | 15 | 34 | −0.6 | 100 | 91 | 8 | 180 |
| 2 (Example 37) | 20 | 28 | 0.1 | 100 | 89 | 10 | 150 |
| 3 (Example 38) | 40 | 16 | 2.6 | 100 | 70 | 15 | 100 |

Note:
Rate of absorption of oxygen . . . same as described in Table 1.

Examples 39 to 44

The procedure of Example 1 was repeated wherein 25 millimoles of TMP, 25 millimoles of a previously prepared crystalline complex, $Li[CuCl_3] \cdot 2H_2O$, and 75 millimoles of LiCl were used and the type of the alcohol used was changed as shown in Table 10.

The results are shown in Table 10.

After the reaction, the reaction mixture separated into a water layer and an alcohol layer. The water layer (catalyst layer), after separation from the alcohol layer, could be recycled and reused.

TABLE 10

| Run No. | Alcohol | Conversion of TMP (%) | Yield of TMBQ (%) | Yield of HMBP (%) | Rate of absorption of oxygen (% per hour) |
|---|---|---|---|---|---|
| 1 (Example 39) | n-Amyl alcohol | 100 | 94 | 6 | 320 |
| 2 (Example 40) | n-Hexyl alcohol | 100 | 93 | 7 | 300 |
| 3 (Example 41) | n-Heptyl alcohol | 100 | 95 | 5 | 350 |
| 4 (Example 42) | n-Octyl alcohol | 100 | 95 | 4 | 370 |
| 5 (Example 43) | n-Nonyl alcohol | 100 | 93 | 7 | 260 |
| 6 (Example 44) | n-Decyl alcohol | 100 | 94 | 6 | 250 |

Note:
Rate of absorption of oxygen . . . same as described in Table 1.

Examples 45 to 49

The procedure of Example 1 was repeated wherein 25 millimoles of TMP, 25 millimoles of a previously prepared crystalline complex, $Li[CuCl_3] \cdot 2H_2O$, and 75 millimoles of LiCl were used, and the amount of n-octanol used was changed as shown in Table 11.

The results are shown in Table 11.

After the reaction, the reaction mixture separated into a water layer and an alcohol layer. The water layer (catalyst layer), after separation from the alcohol layer, could be recycled and reused.

TABLE 11

| Run No. | Amount of n-octanol used (milliliters) | Concentration of TMP in n-octanol (% by weight) | Conversion of TMP (%) | Yield of TMBQ (%) | Yield of HMBP (%) | Rate of absorption of oxygen (% per hour) |
|---|---|---|---|---|---|---|
| 1 (Example 45) | 5 | 45.3 | 100 | 92 | 10 | 100 |
| 2 (Example 46) | 10 | 29.3 | 100 | 92 | 7 | 310 |
| 3 (Example 47) | 20 | 17.2 | 100 | 93 | 6 | 330 |
| 4 (Example 48) | 30 | 10.2 | 100 | 95 | 4 | 295 |
| 5 (Example 49) | 60 | 5.4 | 100 | 85 | 4 | 250 |

Note:
Rate of absorption of oxygen . . . same as described in Table 1.

Examples 50 to 54

The procedure of Example 1 was repeated wherein 25 millimoles of TMP, 25 millimoles of $Li[CuCl_3] \cdot 2H_2O$, and 90 millimoles of LiCl, and the reaction temperature was changed as shown in Table 12.

The results are shown in Table 12.

After the reaction, the reaction mixture separated into a water layer and an alcohol layer. The water layer (catalyst layer), after separation from the alcohol layer, could be recycled and reused.

TABLE 12

| Run No. | Reaction temperature (°C) | Conversion of TMP (%) | Yield of TMBQ (%) | Yield of HMBP (%) | Rate of absorption of oxygen (% per hour) |
|---|---|---|---|---|---|
| 1 (Example 50) | 50 | 100 | 90 | 6 | 250 |
| 2 (Example 51) | 60 | 100 | 95 | 4 | 370 |
| 3 (Example 52) | 70 | 100 | 92 | 7 | 360 |
| 4 (Example 53) | 80 | 100 | 92 | 4 | 340 |
| 5 (Example 54) | 90 | 100 | 91 | 3 | 290 |

Note:

Rate of absorption of oxygen . . . same as described in Table 1.

Comparative Example 4

A mixture of 2.5 grams (25 millimoles) of cuprous chloride, 4.2 grams (100 millimoles) of lithium chloride, and 20 milliliters of methanol was placed in a four-necked flask and stirred to prepare a uniform catalyst solution.

To this catalyst solution was added 3.4 grams (25 millimoles) of TMP, and the reaction was performed under the same conditions as in Example 1. With a lapse of time, a large amount of brown polymeric material deposited on the inner walls of a reactor and a stirrer. In the course of the reaction, absorption of oxygen gradually decreased not to be observed at all. When the oxygen absorption stopped, a large amount of water was added to the reaction mixture while leaving as such the polymeric material deposited. The resulting mixture was extracted with toluene. The extract thus obtained was analyzed by gas chromatography and found to contain only a small amount of TMP. It is believed, therefore, that almost all of TMP reacted was converted into the polymeric material.

Comparative Example 5

A mixture of 25 millimoles of a copper halogeno complex, $Li[CuCl_3] \cdot 2H_2O$, 75 millimoles of LiCl, and 10 milliliters of water was placed in a four-necked flask and stirred to prepare a catalyst solution.

Then, 2.4 grams (25 millimoles) of phenol and 10 milliliters of n-hexanol were introduced into the flask, and the reaction was performed under the same conditions as in Example 1. The time required till the reaction was completed was 4 hours.

The results were as follows:

| | |
|---|---|
| Conversion of phenol | 91.3% |
| Yield of o-chlorophenol | 1.6% |
| Yield of p-chlorophenol | 35.2% |
| Yield of dichlorophenol | 2.0% |

The formation of benzoquinone was not observed. Any other compound could not detected by the gas chromatography analysis.

Example 55

A 200-milliliter four-necked flask was charged with 6.8 grams (50 millimoles) of TMP, 10.6 grams (50 millimoles) of a copper halogeno complex, $Li[CuCl_3] \cdot 2H_2O$, 6.4 grams (150 millimoles) of lithium chloride, LiCl, 20 milliliters of n-hexanol, and 20 milliliters of water. The reaction was performed for 1 hour in the same manner as in Example 1. After the reaction was completed, the reaction mixture was separated into an organic layer and a water layer.

The organic layer and 200 milliliters of benzene were introduced into the separating funnel, and the funnel was shaken to separate a small amount of water from benzene layer including the organic layer.

Thus obtained water was added to the water layer obtained previously. The benzene layer was analyzed by gas chromatography to determine the conversion of TMP and yield of TMBQ. The water layer was returned to the four-necked flask. Then, 6.8 grams of TMP and 20 milliliters of n-hexanol were introduced in the four-necked flask and reacted. This operation was repeated several times and deterioration of the catalyst was examined.

The results are shown in Table 13.

TABLE 13

| Run No. | Number of operations repeated | Conversion of TMP (%) | Yield of TMBQ (%) |
|---|---|---|---|
| 1 | 1 | 100 | 94.8 |
| 2 | 2 | 100 | 94.0 |
| 3 | 3 | 100 | 95.0 |
| 4 | 4 | 100 | 95.1 |
| 5 | 5 | 100 | 94.0 |
| 6 | 6 | 100 | 94.5 |
| 7 | 7 | 100 | 95.2 |
| 8 | 8 | 100 | 94.8 |
| 9 | 9 | 100 | 95.0 |
| 10 | 10 | 100 | 95.0 |

**Claims**

1. A process for producing 2,3,5-trimethylbenzoquinone by oxidizing a substituted phenol with oxygen in the presence of a catalyst, characterized in that it comprises contacting 2,3,6-trimethylphenol with molecular oxygen in a medium of water and an aliphatic alcohol having from 4 to 10 carbon atoms in the presence as a catalyst of a copper halogeno complex represented by the general formula:

$$M_l[Cu(II)_mX_n]_p \qquad (I)$$

wherein

M is an alkali metal or ammonium

Cu(II) is a divalent copper,

X is a halogen atom,

l is an integer of from 1 to 3,

m is 1 or 2,

n is an integer of from 3 to 8,

p is 1 or 2, and

$l+2mp=np$.

2. The process as claimed in claim 1, wherein as a catalyst a mixture of a copper halogeno complex of the formula (I), wherein M, Cu(II), X, l, m, n and p are defined as in claim 1, and an alkali metal halide is used.

3. The process as claimed in claim 1 or 2, wherein the copper halogeno complex is $Li[CuCl_3] \cdot 2H_2O$, $NH_4[CuCl_3] \cdot 2H_2O$, $(NH_4)_2[CuCl_4] \cdot 2H_2O$, $K[CuCl_3]$, $K_2[CuCl_4] \cdot 2H_2O$, $Cs[CuCl_3] \cdot 2H_2O$, $Cs_2[CuCl_4] \cdot 2H_2O$, $Cs_3[Cu_2Cl_7] \cdot 2H_2O$, $Li_2[CuBr_4] \cdot 6H_2O$, $K[CuBr_3]$, $(NH_4)_2[CuBr_4] \cdot 2H_2O$, $Cs_2[CuBr_4]$, or $Cs[CuBr_3]$.

4. The process as claimed in claim 3, wherein the copper halogeno complex is $Li[CuCl_3] \cdot 2H_2O$ or $Li_2[CuBr_4] \cdot 6H_2O$.

5. The process as claimed in claims 1 to 4, wherein the amount of the copper halogeno complex used is from 0,1 to 5 moles per mole of 2,3,6-trimethylphenol.

6. The process as claimed in claims 1 to 5, wherein the aliphatic alcohol is an aliphatic alcohol having from 5 to 10 carbon atoms.

7. The process as claimed in claims 1 to 6, wherein the concentration of 2,3,6-trimethylphenol in the aliphatic alcohol is from 10 to 80% by weight.

8. The process as claimed in claims 1 to 7, wherein the weight ratio of water to the aliphatic alcohol in the medium is from 0,1 to 2.

9. The process as claimed in claims 1 to 8, wherein the concentration of the copper halogeno complex in an aqueous phase of the reaction system is from 20 to 70% by weight.

10. The process as claimed in claim 9, wherein the concentration of the copper halogeno complex in the aqueous phase of the reaction system is from 20 to 60% by weight.

11. The process as claimed in claims 1 to 10 wherein the pH of the aqueous phase containing the copper halogeno complex (catalyst layer) is from 2 to 3.

12. The process as claimed in claim 11, wherein the pH of the aqueous phase containing the copper halogeno complex (catalyst layer) is from 2 to 2,5.

13. The process as claimed in claims 1 to 12, wherein the weight ratio of 2,3,6-trimethylphenol to water in the reaction system is from 0,5 to 10.

14. The process as claimed in claims 1 to 13, wherein the reaction temperature is from 10 to 120°C.

15. The process as claimed in claim 14, wherein the reaction temperature is from 40 to 80°C.

16. The process as claimed in claims 1 to 15, wherein the oxygen partial pressure in the reaction system is from 0,05 to 50 bar ($kg/cm^2$).

17. The process as claimed in claim 16, wherein the oxygen partial pressure in the reaction system is from 0,3 to 1 bar ($kg/cm^2$).

18. The process as claimed in claims 2 to 17, wherein the alkali metal halide is at least one selected from the group consisting of NaCl, LiCl, KCl, CsCl, NaBr, $NH_4Br$, KBr, CsBr, NaI, LiI, KI, and CsI.

19. The process as claimed in claim 18, wherein the alkali metal halide is LiCl.

20. The process as claimed in claims 2 to 19, wherein the molar ratio of alkali metal halide to copper halogeno complex is from 1 to 15.

21. The process as claimed in claim 20, wherein the molar ratio of alkali metal halide to copper halogeno complex is from 1 to 10.

22. The process as claimed in claims 2 to 21, wherein the concentration of the mixture of the copper halogeno complex and alkali metal halide in the aqueous phase of the reaction system is from 20 to 80% by weight.

23. The process as claimed in claim 22, wherein the concentration of the mixture of the copper halogeno complex and alkali metal halide in the aqueous phase of the reaction system is from 20 to 60% by weight.

24. The process as claimed in claims 2 to 23, wherein the pH of the aqueous phase containing the copper halogeno complex and alkali metal halide (catalyst layer) is 2 or less.

25. The process as claimed in claim 24, wherein the pH of the aqueous phase containing the copper halogeno complex and alkali metal halide (catalyst layer) is from 1 to −0,5.

**Patentansprüche**

1. Verfahren zur Herstellung von 2,3,5-Trimethylbenzochinon durch Oxidieren eines substituierten Phenols mit Sauerstoff in Gegenwart eines Katalysators, dadurch gekennzeichnet, daß es umfaßt das Inkontaktbringen von 2,3,6-Trimethylphenol mit molekularem Sauerstoff in einem Medium aus Wasser und einem aliphatischen Alkohol mit 4 bis 10 Kohlenstoffatomen in Gegenwart eines Kupferhalogenkomplexes als Katalysator, dargestellt durch die allgemeine Formel

$$M_l[Cu(II)_mX_n]_p \qquad (I)$$

worin bedeuten:

M ein Alkalimetall oder Ammonium,
Cu(II) zweiwertiges Kupfer,
X ein Halogenatom,
l eine ganze Zahl von 1 bis 3,
m die Zahl 1 oder 2,
n eine ganze Zahl von 3 bis 6,
p die Zahl 1 oder 2, und
$l+2mp=np$.

2. Verfahren nach Anspruch 1, in dem als Katalysator eine Mischung aus einem Kupferhalogenkomplex der Formel (I) worin M, Cu(II), X, l, m, n und p wie in Anspruch 1 definiert sind, und einem Alkalimetallhalogenid verwendet wird.

3. Verfahren nach Anspruch 1 oder 2, worin der Kupferhalogenkomplex ist $Li[CuCl_3]\cdot 2H_2O$, $NH_4[CuCl_3]\cdot 2H_2O$, $(NH_4)_2[CuCl_4]\cdot 2H_2O$, $K[CuCl_3]$, $K_2[CuCl_4]\cdot 2H_2O$, $Cs[CuCl_3]\cdot 2H_2O$, $Cs_2[CuCl_4]\cdot 2H_2O$, $Cs_3[Cu_2Cl_7]\cdot 2H_2O$, $Li_2[CuBr_4]\cdot 6H_2O$, $K[CuBr_3]$, $(NH_4)_2[CuBr_4]\cdot 2H_2O$, $Cs_2[CuBr_4]$ oder $Cs[CuBr_3]$.

4. Verfahren nach Anspruch 3, worin der Kupferhalogenkomplex ist $Li[CuCl_3]\cdot 2H_2O$ oder $Li_2[CuBr_4]\cdot 6H_2O$.

5. Verfahren nach den Ansprüchen 1 bis 4, worin die Menge des verwendeten Kupferhalogenkomplexes 0,1 bis 5 Mol pro Mol 2,3,6-Trimethylphenol beträgt.

6. Verfahren nach den Ansprüchen 1 bis 5, worin der aliphatische Alkohol ein aliphatischer Alkohol mit 5 bis 10 Kohlenstoffatomen ist.

7. Verfahren nach den Ansprüchen 1 bis 6, worin die Konzentration des 2,3,6-Trimethylphenols in dem aliphatischen Alkohol 10 bis 80 Gew.-% beträgt.

8. Verfahren nach den Ansprüchen 1 bis 7, worin das Gewichtsverhältnis von Wasser zu dem aliphatischen Alkohol in dem Medium 0,1 bis 2 beträgt.

9. Verfahren nach den Ansprüchen 1 bis 8, worin die Konzentration des Kupferhalogenkomplexes in einer wäßrigen Phase des Reaktionssystems 20 bis 70 Gew.-% beträgt.

10. Verfahren nach Anspruch 9, worin die Konzentration des Kupferhalogenkomplexes in der wäßrigen Phase des Reaktionssystems 20 bis 60 Gew.-% beträgt.

11. Verfahren nach den Ansprüchen 1 bis 10, worin der pH-Wert der den Kupferhalogenkomplex (Katalysatorschicht) enthaltenden wäßrigen Phase 2 bis 3 beträgt.

12. Verfahren nach Anspruch 11, worin der pH-Wert der den Kupferhalogenkomplex (Katalysatorschicht) enthaltenden wäßrigen Phase 2 bis 2,5 beträgt.

13. Verfahren nach den Ansprüchen 1 bis 12, worin das Gewichtsverhältnis von 2,3,6-Trimethylphenol zu Wasser in dem Reaktionssystem 0,5 bis 10 beträgt.

14. Verfahren nach den Ansprüchen 1 bis 13, worin die Reaktionstemperatur 10 bis 120°C beträgt.

15. Verfahren nach Anspruch 14, worin die Reaktionstemperatur 40 bis 80°C beträgt.

16. Verfahren nach den Ansprüchen 1 bis 15, worin der Sauerstoffpartialdruck in dem Reaktionssystem 0,05 bis 50 bar ($kg/cm^2$) beträgt.

17. Verfahren nach Anspruch 16, worin der Sauerstoffpartialdruck in dem Reaktionssystem 0,3 bis 1 bar ($kg/cm^2$) beträgt.

18. Verfahren nach den Ansprüchen 2 bis 17, worin das Alkalimetallhalogenid mindestens eine Verbindung ist, die ausgewählt wird aus der Gruppe NaCl, LiCl, KCl, CsCl, NaBr, $NH_4Br$, KBr, CsBr, NaI, LiI, KI und CsI.

19. Verfahren nach Anspruch 18, worin das Alkalimetallhalogenid · LiCl ist.

20. Verfahren nach den Ansprüchen 2 bis 19, worin das Molverhältnis von Alkalimetallhalogenid zu Kupferhalogenkomplex 1 bis 15 beträgt.

21. Verfahren nach Anspruch 20, worin das Molverhältnis von Alkalimetallhalogenid zu Kupferhalogenkomplex 1 bis 10 beträgt.

22. Verfahren nach den Ansprüchen 2 bis 21, worin die Konzentration der Mischung aus dem Kupferhalogenkomplex und dem Alkalimetallhalogenid in der wäßrigen Phase des Reaktionssystems 20 bis 80 Gew.-% beträgt.

23. Verfahren nach Anspruch 22, worin die Konzentration der Mischung aus dem Kupferhalogenkomplex und dem Alkalimetallhalogenid in der wäßrigen Phase des Reaktionssystems 20 bis 60 Gew.-% beträgt.

24. Verfahren nach den Ansprüchen 2 bis 23, worin der pH-Wert der den Kupferhalogenkomplex und das Alkalimetallhalogenid (Katalysatorschicht) enthaltenden wäßrigen Phase 2 oder weniger beträgt.

25. Verfahren nach Anspruch 24, worin der pH-Wert der den Kupferhalogenkomplex und das Alkalimetallhalogenid (Katalysatorschicht) enthaltenden wäßrigen Phase 1 bis −0,5 beträgt.

**Revendications**

1. Procédé pour la préparation de la 2,3,5-triméthylbenzoquinone par oxydation d'un phénol substitué, avec de l'oxygène en présence d'un catalyseur, caractérisé en ce qu'il comprend la mise en contact du 2,3,6-triméthylphénol avec de l'oxygène moléculaire, dans un milieu constitué d'eau et d'un alcool aliphatique ayant de 4 à 10 atomes de carbone, en présence d'un catalyseur constitué d'un complexe d'halogène et de cuivre, représenté par la formule générale:

$$M_l[Cu(II)_mX_n]_p \qquad (I)$$

dans laquelle

M est un métal alcalin ou l'ion ammonium,
Cu(II) est le cuivre bivalent,
X est un atome d'halogène,
l est un nombre entier allant de 1 à 3,
m est 1 ou 2,
n est un nombre entier allant de 3 à 8,
p est 1 ou 2, et
$l+2mp=np$.

2. Procédé selon la revendication 1, dans lequel on utilise en tant que catalyseur un mélange d'un complexe d'halogène et de cuivre de formule (I), dans lequel M, Cu(II), X, l, m, n et p sont tels que définis dans la revendication 1, et un halogénure de métal alcalin.

3. Procédé selon la revendication 1 ou 2, dans lequel le complexe d'halogène et de cuivre est $Li[CuCl_3] \cdot 2H_2O$, $NH_4[CuCl_3] \cdot 2H_2O$, $(NH_4)_2[CuCl_4] \cdot 2H_2O$, $K[CuCl_3]$, $K_2[CuCl_4] \cdot 2H_2O$, $Cs[CuCl_3] \cdot 2H_2O$, $Cs_2[CuCl_4] \cdot 2H_2O$, $Cs_3[Cu_2Cl_7] \cdot 2H_2O$, $Li_2[CuBr_4] \cdot 6H_2O$, $K[CuBr_3]$, $(NH_4)_2[CuBr_4] \cdot 2H_2O$, $Cs_2[CuBr_4]$ et $Cs[CuBr_3]$.

4. Procédé selon la revendication 3, dans lequel le complexe d'halogène et de cuivre est $Li[CuCl_3] \cdot 2H_2O$ ou $Li_2[CuBr_4] \cdot 6H_2O$.

5. Procédé selon les revendications 1 à 4, dans lequel la quantité utilisée du complexe d'halogène et de cuivre est de 0,1 à 5 moles, par mole de 2,3,6-triméthylphénol.

6. Procédé selon les revendications 1 à 5, dans lequel l'alcool aliphatique est un alcool aliphatique ayant de 5 à 10 atomes de carbone.

7. Procédé selon les revendications 1 à 6, dans lequel la concentration du 2,3,6-triméthylphénol dans l'alcool aliphatique est de 10 à 80% en poids.

8. Procédé selon les revendications 1 à 7, dans lequel le rapport pondéral de l'eau à l'alcool aliphatique dans le milieu est de 0,1 à 2.

9. Procédé selon les revendications 1 à 8, dans lequel la concentration du complexe d'halogène et de cuivre dans la phase aqueuse du système réactionnel est de 20 à 70% en poids.

10. Procédé selon la revendication 9, dans lequel la concentration du complexe d'halogène et de cuivre dans la phase aqueuse du système réactionnel est de 20 à 60% en poids.

11. Procédé selon les revendications 1 à 10, dans lequel le pH de la phase aqueuse contenant le complexe d'halogène et de cuivre (phase contenant le catalyseur) est de 2 à 3.

12. Procédé selon la revendication 11, dans lequel le pH de la phase aqueuse contenant le complexe d'halogène et de cuivre (phase contenant le catalyseur) est de 2 à 2,5.

13. Procédé selon les revendications 1 à 12, dans lequel le rapport pondéral du 2,3,6-triméthylphénol à l'eau dans le système réactionnel est de 0,5 à 10.

14. Procédé selon les revendications 1 à 13, dans lequel la température de la réaction est de 10 à 120°C.

15. Procédé selon la revendication 14, dans lequel la température de la réaction est de 40 à 80°C.

16. Procédé selon les revendications 1 à 15, dans lequel la pression partielle de l'oxygène dans le système réactionnelle est de 0,05 à 50 bars ($kg/cm^2$).

17. Procédé selon la revendication 16, dans lequel la pression partielle de l'oxygène dans le système réactionnel est de 0,3 à 1 bar ($kg/cm^2$).

18. Procédé selon les revendications 2 à 17, dans lequel l'halogénure de métal alcalin est au moins un halogénure choisi parmi NaCl, LiCl, KCl, CsCl, NaBr, $NH_4Br$, KBr, CsBr, NaI, LiI, KI et CsI.

19. Procédé selon la revendication 18, dans lequel l'halogénure de métal alcalin est LiCl.

20. Procédé selon les revendications 2 à 19, dans lequel le rapport molaire de l'halogénure de métal alcalin au complexe d'halogène et de cuivre est de 1 à 15.

21. Procédé selon la revendication 20, dans lequel le rapport molaire de l'halogénure de métal alcalin au complexe d'halogène et de cuivre est de 1 à 10.

22. Procédé selon les revendications 2 à 21, dans lequel la concentration du mélange du complexe d'halogène et de cuivre et de l'halogénure de métal alcalin dans la phase aqueuse du système réactionnel est de 20 à 80% en poids.

23. Procédé selon la revendication 22, dans lequel la concentration du mélange du complexe d'halogène et de cuivre et de l'halogénure de métal alcalin dans la phase aqueuse du système réactionnel est de 20 à 60% en poids.

24. Procédé selon les revendications 2 à 23, dans lequel le pH de la phase aqueuse contenant le complexe d'halogène et de cuivre et l'halogénure de métal alcalin (phase contenant le catalyseur) est égal ou inférieur à 2.

25. Procédé selon la revendication 24, dans lequel le pH de la phase aqueuse contenant le complexe d'halogène et de cuivre et l'halogénure de métal alcalin (phase contenant le catalyseur) est de 1 à −0,5.

FIG. 1

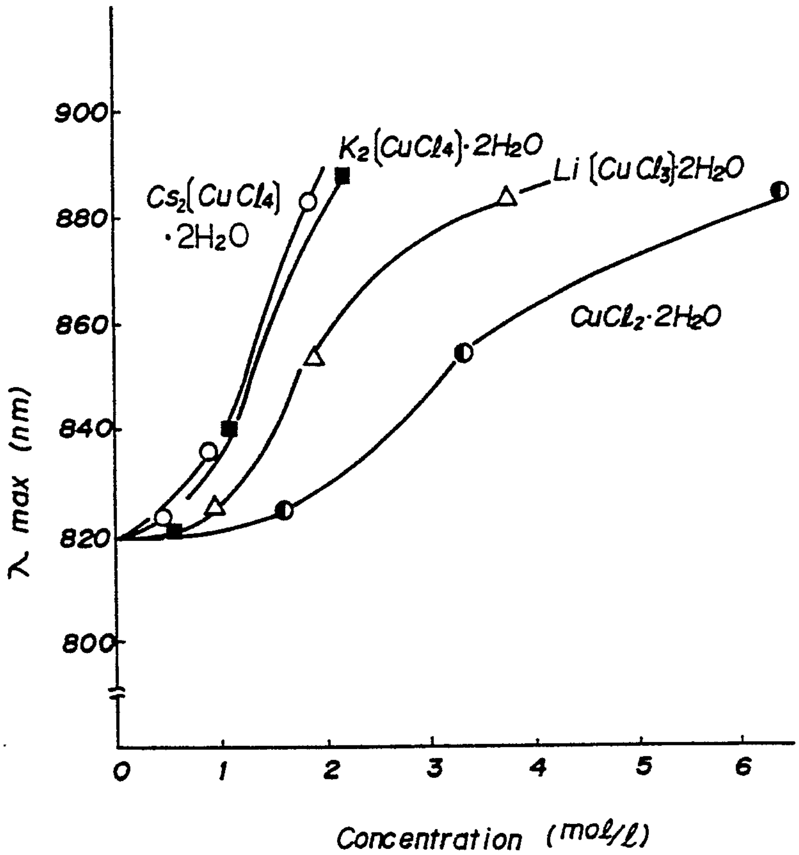
900
880
860
840
820
800
λ max (nm)
K2(CuCl4)·2H2O
Cs2(CuCl4)
·2H2O
Li (CuCl3)2H2O
CuCl2·2H2O
0
1
2
3
4
5
6
Concentration (mol/l)

FIG. 2

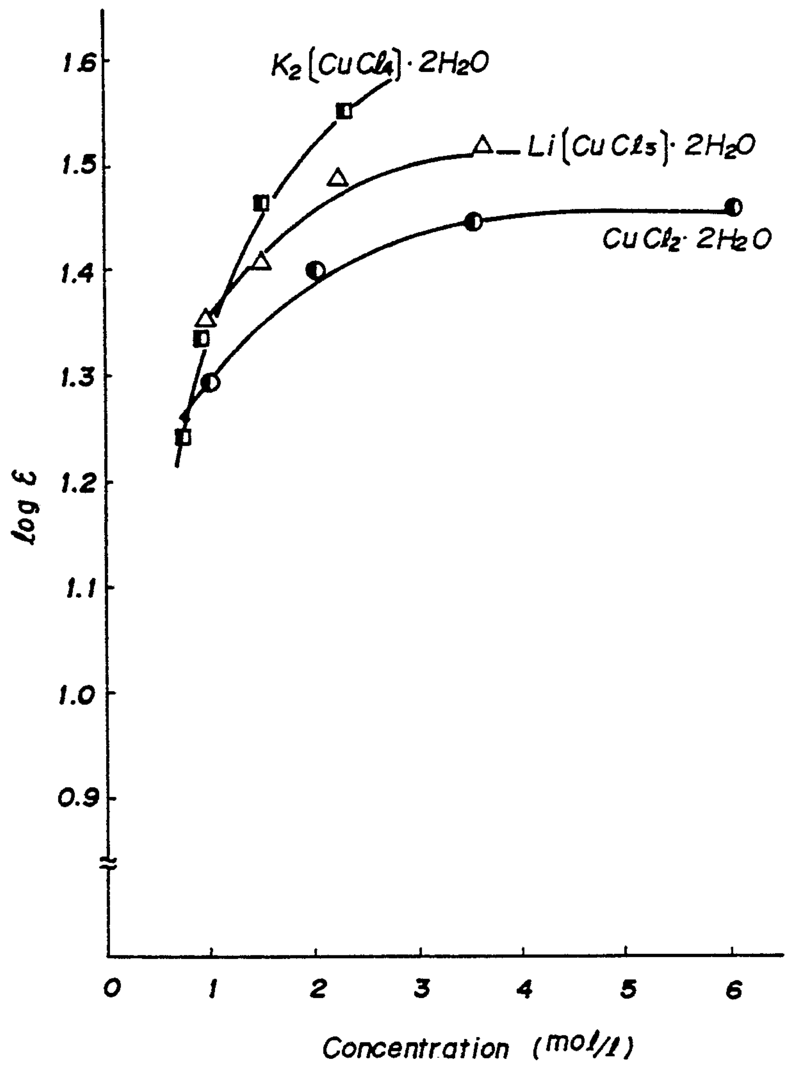

K2 [CuCl4] · 2H2O
Li [CuCl3] · 2H2O
CuCl2 · 2H2O
1.6
1.5
1.4
1.3
1.2
1.1
1.0
0.9
log ε
0
1
2
3
4
5
6
Concentration (mol/l)

FIG. 3